# EUROPEAN PATENT SPECIFICATION

(11) **EP 0 499 401 B1**
(45) Date of publication and mention of the grant of the patent: **26.04.1995**
(21) Application number: 92300992.2
(22) Date of filing: 06.02.1992
(51) Int. Cl.: A61M 39/04

(54) **Resealable sampling port**
Wiederverschliessbares Port zur Entnahme von Proben
Port de prélèvement d'échantillons refermable

(30) Priority: 14.02.1991 GB 9103122; 03.07.1991 GB 9114341
(43) Date of publication of application: 19.08.1992
(73) Proprietor: SMITHS INDUSTRIES PLC, London NW11 8DS (GB)
(72) Inventor: Wallace, Henry George, Frinton-on-Sea (GB)
(74) Representative: Stebbing, Peter John Hunter

(56) References cited:
- EP-A- 0 198 962
- EP-A- 0 369 314
- WO-A-90/02579

## Description

The present invention relates to resealable sampling ports and injection sites, particularly for biological materials. Sampling ports and section sites can be used to extract or deliver a fluid from, or to, a conduit without introducing microorganisms into the conduit and without allowing the contents of the conduit to led after a syringe has been withdrawn. The port or site may be swabbable either by being flush with the housing or by being accessible in a reasonable manner for example by cotton wool. Swabbing is necessary to disinfect the external surface of the diaphragm to prevent contamination of the conduit contents and also to stop growth of bacteria in residual fluid resulting from extraction of a sample of the conduit contents.

Such arrangements are known to be provided with a resealable diaphragm which may optionally be pre-slit. Whereas resealable diaphragms are satisfactory for use with a needle, since the puncture in the resealable septum or diaphragm is small, there are problems associated with utilising a syringe having a cone for introduction through a pre-slit resealable diaphragm since the external bore of the cone is much larger than that of the needle and presents a challenge to the resealability of the diaphragm.

It is obviously desirable to use syringes for sampling where possible because their use avoids the problem of needlestick and because syringes can remove and deliver much greater quantities of liquid more rapidly than needles. Further, the cost of replacement needles is saved, and the problem of needle disposal is reduced.

Conical resealing valves for cooperation with Luer-type syringes are known in the art. One such example was made by Rusch in Germany in 1967 and comprised a resealable septum in a bore which was adapted to accommodate the syringes. Such devices are more suitable where the septum does not require to be disinfected for example by swabbing. Also they cannot prevent flash-back of fluid into a pre-entry portion of the valve. Foley urological catheters, commonly have a pre-slit diaphragm to accept a Luer syringe cone.

WO89/06553 (Baxter) provides a shaped pre-slit resealable bung retained in an annular recess coaxial therewith. The recess is a precise fit in the radial direction and is provided with an annular space into which bung material may be forced as the syringe cone slides through the slit in use. Since the syringe cone has a taper increasing force upon the syringe acts to progressively deform the bung during interengagement. However, the design of the bung is such that the arrangement cannot be used with a standard Luer taper due to the inbuilt radial compression exerted on the diaphragm by the housing assembly. Thus a special adaptor cannula must be fitted to the syringe before engagement with the bung. This arrangement thus relies upon radial deformation of the bung into the annular space to open and reseal repeatedly. The arrangement as shown in Baxter is therefore unsuited to smaller sampling ports, and even with pre-slit injection sites requires a relatively high interengagement pressure.

One of the problems with the withdrawal of blood by a needle or syringe cone has been that blood tends to be left, after withdrawal of the needle or syringe cone on the housing or exterior of the needle or cone.

This has been addressed in Baxter WO89-O6553 by swabbing the top of the septum and the cone as appropriate and with this in mind the target area of the septum is convex. Another approach to this problem has been in discussed in GB-A-1444210 which provides a bung with a bore terminating in a resealable septum and being provided with an inwardly directed annulus adapted to contact the needle to wipe the blood from the exterior faces thereof. In an embodiment of the present invention this is improved by the use of (a) an arrangement for ensuring that the withdrawal of the cone temporarily increases the wiping pressure; and (b) by providing a double wiping action.

One object of the present invention is thus to provide an annulus and diaphragm assembly for cooperation with a housing of a sampling port or injection site, which assembly allows for the easy introduction of a syringe cone, while ensuring a good biological seal between the cone and the assembly. A further object is to provide an annulus and diaphragm assembly wherein a cantilever action is used to apply a point loading against the exterior of the cone as it is either introduced or withdrawn from the injection site or sampling port.

Another object is to provide an injection site incorporating a double cantilever effect for sealing purposes which is particularly, although not exclusively, suited to an injection site.

Another object for the invention is to provide a sampling port, or injection site, in which sealing can be effected between the cone and the annulus and diaphragm assembly without the undue pressure and deformation of the diaphragm previously required.

Another object of the invention is to provide an annulus an diaphragm assembly which in its at-rest condition forms a laterally slidable seal within the housing of a port or injectable site, which seal is reinforced by cantilever action between the housing and the assembly on introduction, and optionally withdrawal, of a syringe cone.

Accordingly rather than cause a radial deformation of the septum as required in WO-A-89/06553, the present invention provides in essence means whereby a cantilever action is employed to seal the diaphragm assembly while the syringe cone is introduced and to reseal the said assembly as the cone is removed.

Sealing against the syringe cone is achieved at least in part by a cantilever action of the diaphragm on entry of the cone. This approach makes possible a light and compact port and/or an effective injection site.

From WO-A-90/02579 it is known to provide a resealable sampling port or an injection site for use with a syringe terminating in a blunt syringe cone, said port being formed with a housing including a conduit sealed by a resealable diaphragm, and a sealing annulus juxtaposed to, and interacting with, the diaphragm to form an annulus and diaphragm assembly.

The present invention is characterised in that the assembly forms a firm sliding seal in the housing in the lateral direction relative to the insertion direction of the syringe and in that arrangement of the housing and the assembly is such that the action of the syringe cone as it passes into the assembly both generates a radial distension and a cantilever action of the assembly which serves to seal the diaphragm and annulus in the housing.

In the preferred embodiments, the resealable diaphragm and annulus assembly comprises a generally planar portion forming the diaphragm having a pre-slit channel disposed generally centrally thereof; and coaxial of said channel an upstanding or downwardly depending sealing annulus, said annulus having a bore approximated to the external dimension of a syringe cone with which it is to be used; said diaphragm being adapted for radial distension and being capable of flexing about the pre-slit channel to urge the adjacent portion of the sealing annulus into abutment with the cone in use.

Preferably the syringe cone in accordance with the present invention has a defined taper and may for example be a standard Luer syringe cone having a 6% taper. The material to be withdrawn, sampled or introduced, is most preferably a biological material such as blood or urine; or a biologically active agent such as for example insulin.

The resealable diaphragm should be capable of a slight centering action if possible relative to the housing to accommodate inaccuracy in locating the syringe cone in the diaphragm.

In a preferred form of the invention the annulus and the diaphragm are integrally formed. In such an arrangement the peripheral portions of the integral annulus and diaphragm units may be provided with a central channel thereby to define outwardly directed portions. The annulus and diaphragm are preferably loosely retained by the housing perpendicular to the axis of entry of the cone, thereby to accommodate an eccentric cone. It is also preferred that the opening of a sampling port should be in the form of a bowl or well having a diameter less than that of the diaphragm to provide a cantilever point.

In a preferred form of the invention the diaphragm is pre-slit and the annulus has an at-rest diameter slightly less than that of a coaxial bore in a retaining cap. By this means the act of passing the syringe cannula through the bore in the cap until said syringe is in contact therewith, also seals the annulus against the syringe cone; which seal is reinforced by the action of the diaphragm on the sealing annulus.

In such an arrangement it will preferably be arranged that the displacement of annulus on interengagement of the syringe cone with the annulus also serves to seal the annulus and the diaphragm against the housing and the retaining cap.

The invention will now be described, by way of illustration only, with reference to the accompanying drawings wherein:-
Figure 1 shows a vertical cross section through a sampling port in accordance to the present invention;
Figure 2 shows a side view from above of the port as shown in Figure 1;
Figure 3 shows a side view of the Figure 1 in use;
Figure 4 shows in vertical cross-section the arrangement of Figure 1 applied to an injection site;
Figure 5 shows a vertical cross-section of another form of diaphragm and annulus assembly of Figure 4;
Figure 6 shows in vertical cross-section a further form of a diaphragm and annulus assembly; and
Figure 7 shows in vertical cross-section an arrangement of Figure 6 in use with a syringe cone.

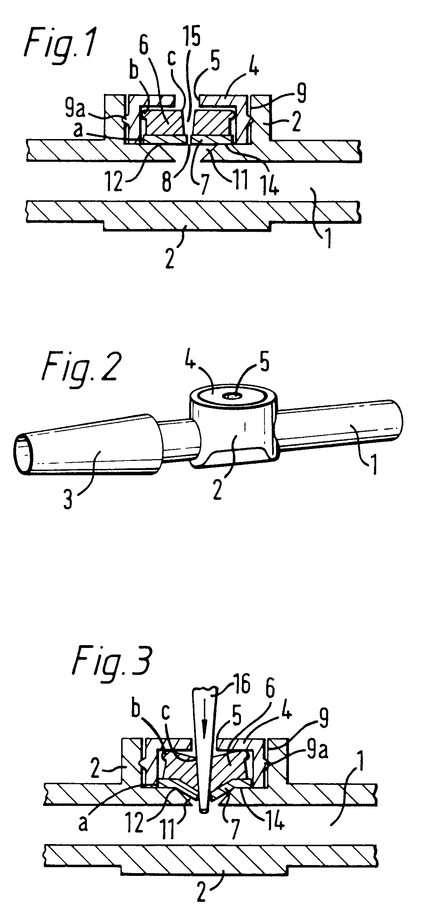

With reference first to Figure 2 a cylindrical bore defining a conduit 1 is provided at its remote end with a connector portion 3 of standard type. Disposed about the conduit 1 is a generally annular sampling port 2, provided on its upper face with a retaining cap 4 provided with a retaining cap bore 5 adapted in use to accommodate a Luer syringe cone (16).

Turning now to Figure 1. The conduit 1 is provided on its upper face with an upstanding portion forming the sampling port 2. The inner face of the sampling port 2 is provided with a channel 9. The circular floor of the sampling port 2 is depressed to form a well 11 which forms a generally bow shape in cross-section so as to provide a connection through into the conduit 1. The well 11 terminates at a cantilever point 12. The floor 14 extends radially outwardly from the point 12 towards the inner periphery of the sampling port 2.

The sampling port 2 also accommodates a coaxial retaining cap 4 provided on its outer face with a rib 9a for cooperation with channel 9 to lock the said cap into the port 2. Alternatively, the retaining cap may be welded or bonded in situ. The retaining cap 4 is provided with a retaining cap bore 5 which is generally coaxial about the well 11. Retained in the space formed by the retaining cap 4 and the floor 14 is a diaphragm and annular seal assembly, wherein the diaphragm is designated 7 and the annular seal designated 6. It will be noted that the annular seal 6 is provided with a internal blind bore 15 terminating at its lower most end in the diaphragm 7. The diaphragm 7 is provided with a pre-slit channel 8. It will be appreciated that in its normal at-rest condition the annular seal and diaphragm assembly is a firm sliding seal between the lower face of the retaining cap 4 and the floor 14. A strong pressure exerted on a side of the annular seal 6 will allow a slight lateral movement of the assembly to a limited extent, as in the normal at-rest condition there is a firm seal between the conduit and the exterior.

In use as shown in Figure 3 a cone of a Luer syringe 16 is passed through the bore 5 and into the annular seal 6. Since such a Luer cone has a 6% taper initially it will pass through the annular seal 6 with a minimum deformation. Further urging of the cone towards to the conduit 1 causes the tip of the cone to enter the pre-slit channel 8 and deform the diaphragm downwardly into the well 11. At the same time the extra penetration causes the outer periphery of the cone to come into contact with the edges of the retaining cap bore 5.

The effect of this arrangement is to force the center of the diaphragm downwardly about the cantilever point 12. Because of the bulk of the cone, the diaphragm is distended outwardly in a radial direction whereby the point (a) is forced into compression with the inner face of the wall of the retaining cap. Further, the cantilever moment about the point 12 forces the point (b) of the annular seal into compression against the underside of the retaining cap 4, while because of the geometry of the bore in the annular seal a point (c) is forced more firmly against the wall of the cone.

Because the annular seal and the diaphragm are formed of a biologically acceptable resilient material such as natural rubber, the cone when fully introduced is locked into the sampling port by means of its contact with the bore 5 in the retaining cap 4, the point (c) of the annular seal and because it is distended through the pre-slit channel 8. In this condition biological materials flowing in the conduit 1 can be extracted or additional components added to the said conduit as desired.

Withdrawal of the cone from the pre-slit channel 8 allows the assembly to return to its at-rest condition as shown in Figure 1 without allowing any leakage and without any collateral damage to the diaphragm 7 itself.

When the tip of the cone 16 has been sampling blood, for example, it is desirable that as much blood as possible is removed from the cone as the cone exits the sampling port. This arrangement achieves this with facility. As cone 16 is withdrawn, the diaphragm 7 is urged upwardly until the upper face of the annulus 6 abuts the underside of the cap 4. At this point the cone is relatively withdrawn from the annulus and diaphragm assembly. Because of the shape of the bore in the annulus sliding contact between the cone and the annulus is not only maintained but is also slightly increased as the cone is withdrawn. Thus blood is wiped off the cone and particularly from the tip thereof as it passes through the diaphragm and annulus assembly, in part at least because of this "reverse cantilever" action.

It will be appreciated that in the arrangement as shown the diaphragm and the annular seal are made of the same material and are integral. However, a device can equally well be made where the annular seal and the diaphragm are made of different biologically acceptable forms of material and are separate; said separation may be physical, or may be via an adhesive layer if appropriate.

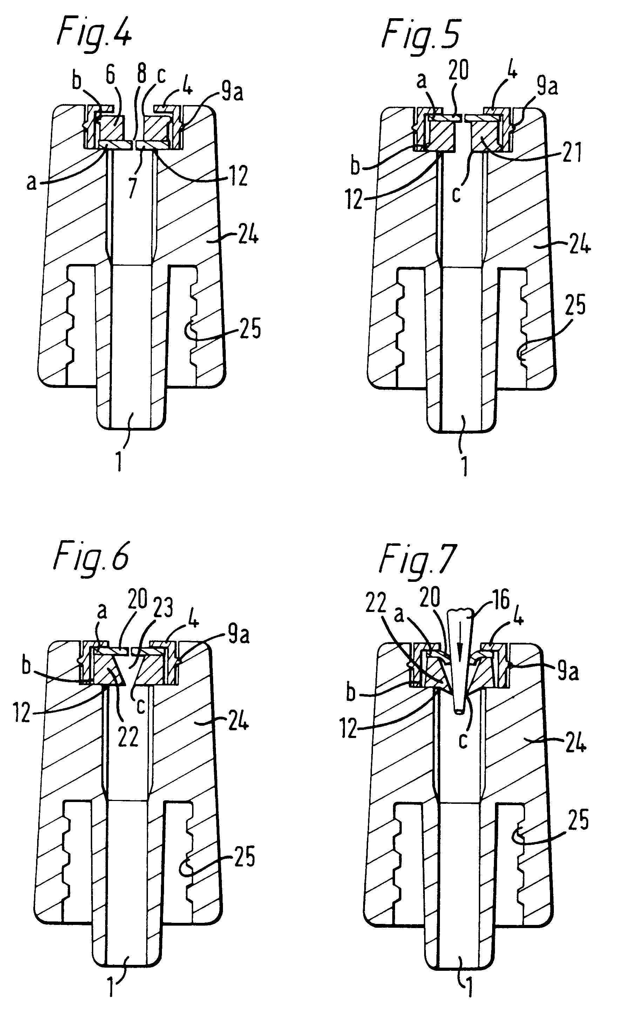

Figures 4 to 7 relate to different forms of an injectable site. In Figures 4 to 7 injection sites formed with a female Luer lock screw thread are shown. A male Luer lock screw thread can be substituted.

With specific reference to Figure 4 there is provided an annular seal 6 and a resealable diaphragm 7 substantially as shown in Figure 1 and which operates substantially in accord with the arrangement shown in Figure 3. This modus operandi has been already described with reference to Figure 3 and will not be described further with the exception that it will be noted that the conduit 1 is coaxial with the annular seal and resealable diaphragm assembly rather than disposed perpendicular thereto. The housing 24 is formed with a cantilever point 12 substantially as shown in Figure 1. The housing 24 is also provided with coaxial Luer screw threads 25 of known type.

While the arrangement of Figure 4 works satisfactorily it presents a problem that the surface of the diaphragm 7 is not readily swabbable for the purposes of sterilization. Accordingly, in the arrangement shown in Figure 5 the annular seal 6 and the resealable diaphragm 7 are reversed such that the resealable diaphragm 20 is positioned superior to the lower sealing annulus 20. It will be noted that the introduction of a cone 16 into the resealable diaphragm 20 causes a cantilever action of the diaphragm and a deformation of the annulus outwardly.

The arrangement of Figure 6 presents an improvement of the arrangement of Figure 5 in that the annular seal 22 is formed with a fusto-conical space 23. This arrangement, shown operational detail in Figure 7, provides for a double seal against the cone of the syringe 16 as it is introduced into the injectable site. On withdrawal of the cone 16 the initial action is to increase the sealing pressure between the diaphragm 20 and the annular seal 22 before a sliding action is engendered. The double seal as shown in Figure 7 is particular useful in preventing contaminants from being left on the cone after withdrawal.

By use of the present arrangement it has been found that a large number of introductions of a Luer cone of standard dimensions can be accommodated without degrading the natural rubber diaphragm and allowing organism entry. The invention therefore provides a novel resealable sampling port and/or an injection site and a combined resealable diaphragm and sealing annulus for use therewith. Further the retaining cap provides a bore to lock the taper of a syringe cone when the cone is fully inserted into the port when the device is utilised for a resealable sampling port.

## Claims

1. A resealable sampling port (2) or an injection site (24) for use with a syringe terminating in a blunt syringe cone (16), said port being formed with a housing including a conduit (1) sealed by a resealable diaphragm (7), and a sealing annulus (6) juxtaposed to, and interacting with, the diaphragm to form an annulus and diaphragm assembly, characterised in that the assembly forms a firm sliding seal in the housing in the lateral direction relating to the insertion direction of the syringe and in that arrangement of the housing and the assembly is such that the action of the syringe cone (16) as it passes into the assembly both generates a radial distension and a cantilever action of the assembly which serves to seal the diaphragm and annulus in the housing.

2. A port or site according to Claim 1 wherein the diaphragm (7) is downstream of the annulus (6).

3. A port or site according to Claim 1 wherein the diaphragm (20) is upstream of the annulus (21).

4. A port or site as claimed in any preceding Claim wherein the housing, and the annulus and diaphragm assembly, are constructed and arranged such that withdrawal of the cone (16) from the diaphragm (7;20) exerts an increased pressure between the cone, and the diaphragm and annulus assembly, to seal the same against the withdrawing cone.

5. A port or site according to any of claims 1 to 4 wherein the conduit comprises a well (11) with a lateral dimension less than that of the diaphragm (7) and situated immediately downstream of the annulus and diaphragm assembly.

6. A port or site according to any preceding Claim wherein the annulus and diaphragm assembly is integral and is formed of the same or different biologically acceptable resilient materials.

7. A port or site according to any preceding Claim wherein the peripheral portions of an integral annulus and diaphragm unit are respectively provided with a radially directed rib portion (a.b).

8. A port or site according to any proceeding Claim wherein the diaphragm is pre-slit, and wherein the annulus has an at-rest diameter slightly less than that of a coaxial bore in a retaining cap portion (4) of the housing.

9. A port or site according to any of Claims 1 to 8 wherein the displacement of the sealing annulus also acts to seal the annulus against the housing.

10. A port or site according to any preceding Claim wherein the housing is formed with the retaining cap (4) having an axial bore of a diameter lees than the maximum diameter of the cone (16) with which it is to be used.

11. A port on site according to any preceding claim, wherein the resealable diaphragm and annulus assembly (6,7:20,21,22) comprises a generally planar portion forming the diaphragm (7;20) having a pre-slit channel (8) disposed generally centrally thereof; and coaxial of said channel an upstanding or downwardly depending sealing annulus (6;21), said annulus having a bore (15;23) approximated to the external dimension of a syringe cone (16) with which it is to be used; said diaphragm being adapted for radial distension and being capable of flexing about the pre-slit channel to urge the adjacent portion of the sealing annulus into abutment with the cone in use.

12. A port or site according to Claim 11 wherein the interior faces of the sealing annulus converge to form a frusto-conical annular space (23) having its minimum diameter (c) remote from the diaphragm (20).

## Patentansprüche

1. Wiederverschließbarer Probenahme-Durchlaß (2) oder Injektionsstelle (24) zum Einsatz in verbindung mit einer Spritze, welche mit einem stumpfen Spritzenkonus (16) endet, wobei der Durchlaß von einem Gehäuse gebildet wird, welcher eine Leitung (1) umfaßt, die mittels einer wiederverschließbaren Membrane (7) dicht verschlossen ist, und einen Dichtringkörper (6) umfaßt, welcher angrenzend hierzu angeordnet ist und mit der Membrane zusammenarbeitet, um eine Ring- und Membrananordnung zu bilden, **dadurch gekennzeichnet**, daß die Anordnung eine fest Gleitdichtung im Gehäuse in Querrichtung bezüglich der Einführungsrichtung der Spritze bildet, und daß das Gehäuse und die Anordnung derart ausgelegt sind, daß die Wirkung des Spritzenkonus (16) beim Durchgang in der Anordnung sowohl eine radiale Aufweitung als auch eine Tragarmwirkung der Anordnung erzeugt, welche dazu beitragen, daß die Membrane und der Ringkörper im Gehäuse dicht abgeschlossen werden.

2. Durchlaß oder Stelle nach Anspruch 1, bei der die Membrane (7) stromabwärts von dem Ringkörper (6) angeordnet ist.

3. Durchlaß oder Stelle nach Anspruch 1, bei der die Membrane (20) stromaufwärts von dem Ringkörper (21) angeordnet ist.

4. Durchlaß oder Stelle nach einem der vorangehenden Ansprüche, bei der das Gehäuse und die Ringkörper- und Membrananordnung derart beschaffen und ausgelegt sind, daß beim Abziehen des Konus (16) von der Membran (7; 20) ein verstärkter Druck zwischen dem Konus und der Membran- und Ringkörperanordnung aufgebracht wird, um diese entgegen der Abzugsbewegung des Konus abzudichten.

5. Durchlaß oder Stelle nach einem der Ansprüche 1 bis 4, bei der die Leitung einen umschlossenen, freien Raum (11) mit Querabmessungen aufweist, welche kleiner als jene der Membrane (7) sind, und der unmittelbar stromabwärts von der Ringkörper- und Membrananordnung liegt.

6. Durchlaß oder Stelle nach einem der vorangehenden Ansprüche, bei der die Ringkörper- und Membrananordnung integral ausgebildet ist und aus dem gleichen oder einem unterschiedlichen biologisch verträglichen, federnd nachgiebigen Material hergestellt ist.

7. Durchlaß oder Stelle nach einem der vorangehenden Ansprüche, bei der die Umfangsteile einer integralen Ringkörper- und Membraneinheit jeweils mit einem radialweisenden Rippenabschnitt (a, b) versehen sind.

8. Durchlaß oder Stelle nach einem der vorangehenden Ansprüche, bei der die Membrane vorgeschlitzt ist, und bei der der Ringkörper einen Grunddurchmesser hat, der geringfügig kleiner als jener einer koaxialen Bohrung in einem Haltekappenteil (4) des Gehäuses ist.

9. Durchlaß oder Stelle nach einem der Ansprüche 1 bis 8, bei der die Verschiebung des Dichtringkörpers auch bewirkt, daß der Ringkörper gegenüber dem Gehäuse abgedichtet wird.

10. Durchlaß oder Stelle nach einem der vorangehenden Ansprüche, bei dem das Gehäuse mit einer Haltekappe versehen ist, welche eine axiale Bohrung mit einem Durchmesser hat, welcher kleiner als der maximale Durchmesser des Konus (16) ist, in Verbindung mit welchem sie zum Einsatz kommt.

11. Durchlaß oder Stelle nach einem der vorangehenden Ansprüche, bei der die wiederverschließbare Membran- und Ringkörperanordnung (6, 7; 20, 21, 22) einen im allgemeinen planaren Abschnitt aufweist, welcher die Membrane (7; 20) bildet, welche einen vorgeschlitzten Kanal (8) hat, der im allgemeinen zentrisch an dieser vorgesehen ist; und koaxial zu dem Kanal ein aufrechtstehender oder sich hängend nach unten erstreckender Dichtringkörper (6; 21) vorgesehen ist, der Ringkörper eine Bohrung (15; 23) hat, welche in etwa den Außenabmessungen eines Spritzenkonus (16) entspricht, mit dem dieser beim Einsatz zusammenarbeitet; und daß die Membrane derart ausgelegt ist, daß man eine radiale Aufweitung erhält und eine Biegbarkeit um den vorgeschlitzten Kanal hat, um den benachbarten Abschnitt des Dichtringkörpers in Anlageberührung an dem Konus im Gebrauchszustand anzudrücken.

12. Durchlaß oder Stelle nach Anspruch 11, bei der die Innenflächen des Dichtringkörpers konvergierend verlaufen, um einen kegelstumpfförmigen Ringraum (23) zu bilden, welcher seinen kleinsten Durchmesser (c) entfernt von der Membrane (20) hat.

## Revendications

1. Embouchure d'echantillonnage (2) apte à se refermer hermétiquement ou site d'injection (24), en vue d'une utilisation avec une seringue se terminant en un cône de seringue émoussé (16), ladite embouchure étant formée avec un logement incluant un conduit (1) hermétiquement fermé par un diaphragme (7) apte à se refermer hermétiquement, et un anneau d'étanchéité (6) juxtaposé à, et interagissant avec, le diaphragme afin de former un ensemble anneau et diaphragme, caractérisé par le fait que l'ensemble forme un joint d'étanchéité coulissant, ferme, dans le logement dans la direction latérale par rapport à la direction d'introduction de la seringue, et par le fait que la disposition du logement et de l'ensemble est telle que l'action du cône de seringue (16), alors qu'il passe dans l'ensemble, produit à la fois une dilatation radiale et une action de porte-à-faux de l'ensemble qui sert à sceller hermétiquement le diaphragme et l'anneau dans le logement.

2. Embouchure ou site selon la revendication 1, dans lequel le diaphragme (7) est en aval de l'anneau (6).

3. Embouchure ou site selon la revendication 1, dans lequel le diaphragme (20) est en amont de l'anneau (21).

4. Embouchure ou site selon l'une des revendications précédentes, dans lequel le logement, et l'ensemble anneau et diaphragme, sont construits et disposés de telle sorte que le retrait du cône (16) à partir du diaphragme (7 ; 20) exerce une pression accrue entre le cône, et l'ensemble diaphragme et anneau, pour sceller hermétiquement ce dernier contre le cône se retirant.

5. Embouchure ou site selon l'une des revendications 1 à 4, dans lequel le conduit comprend un puits (11) ayant une dimension latérale inférieure à celle du diaphragme (7), et situé immédiatement en aval de l'ensemble anneau et diaphragme.

6. Embouchure ou site selon l'une des revendications précédentes, dans lequel l'ensemble anneau et diaphragme est d'un seul tenant et est formé de matières élastiques, biologiquement acceptables, identiques ou différentes.

7. Embouchure ou site selon l'une des revendications précédentes, dans lequel les parties périphériques d'une unité anneau et diaphragme d'un seul tenant sont dotées respectivement d'une partie de nervure orientée radialement (a.b).

8. Embouchure ou site selon l'une des revendications précédentes, dans lequel le diaphragme est préfendu, et dans lequel l'anneau a un diamètre au repos légèrement inférieur à celui d'un alésage coaxial dans la partie de capuchon de retenue (4) du logement.

9. Embouchure ou site selon l'une des revendications 1 à 8, dans lequel le déplacement de l'anneau d'étanchéité agit également pour sceller hermétiquement l'anneau contre le logement.

10. Embouchure ou site selon l'une des revendications précédentes, dans lequel le logement est formé avec le capuchon de retenue (4) ayant un alésage axial d'un diamètre inférieur au diamètre maximum du cône (16) avec lequel il doit être utilisé.

11. Embouchure ou site selon l'une des revendications précédentes, dans lequel l'ensemble diaphragme apte à se refermer hermétiquement et anneau (6, 7 ; 20, 21, 22) comprend une partie généralement plane formant le diaphragme (7 ; 20) présentant un canal préfendu (8) disposé généralement au centre de celle-ci ; et coaxialement avec ledit canal un anneau d'étanchéité (6 ; 21) dressé ou pendant vers le bas, ledit anneau ayant un alésage (15 ; 23) de dimension s'approchant de la dimension externe d'un cône de seringue (16) avec lequel il doit être utilisé ; ledit diaphragme étant adapté en vue d'une dilatation radiale et étant capable de fléchir autour du canal préfendu pour pousser la partie adjacente de l'anneau d'étanchéité en butée avec le cône lors de l'utilisation.

12. Embouchure ou site selon la revendication 11, dans lequel les faces intérieures de l'anneau d'étanchéité convergent pour former un espace annulaire tronconique (23) ayant son diamètre minimum (c) éloigné du diaphragme (20).
